# EUROPEAN PATENT APPLICATION

(11) **EP 4 628 506 A1**
(43) Date of publication of application: **08.10.2025**
(21) Application number: 23896818.4
(22) Date of filing: 29.11.2023
(51) Int. Cl.: C07K 16/28, C07K 19/00, A61K 51/10, A61P 35/00, A61K 101/02, A61K 103/00

(54) **PREPARATION METHOD FOR AND USE OF CD70-SPECIFIC INTEGRATED DIAGNOSIS AND TREATMENT MOLECULAR IMAGING PROBE**

(30) Priority: 30.11.2022 CN 202211527715
(71) Applicant: Renji Hospital, Shanghai Jiao Tong University School of Medicine, Shanghai 200001 (CN)
(72) Inventor: WEI, Weijun, Shanghai 200001 (CN); WU, Qianyun, Shanghai 200001 (CN); LIU, Jianjun, Shanghai 200001 (CN)
(74) Representative: Plavsa, Olga
(86) International application number: PCT/CN2023/134962
(87) International publication number: WO 2024/114674

(57) **Abstract**

Provided in the present invention are a preparation method for and the use of a CD70-specific integrated diagnosis and treatment molecular imaging probe. The probe includes a tumor targeting group and radionuclides. The tumor targeting group is selected from a CD70-specific nanoantibody or a CD70-specific nanoantibody fusion protein, wherein the CD70-specific nanoantibody is B3 or B6, the amino acid sequences thereof being shown as SEQ ID NO. 1 and SEQ ID NO. 3, and the CD70 specific nanoantibody fusion protein is ABDB3 or ABDB6, the amino acid sequences thereof being shown as SEQ ID NO. 5 and SEQ ID NO. 7. The present invention achieves non-invasive visualization of human CD70 molecular expression and thus achieves noninvasive diagnosis of renal cell carcinoma. The probe has the advantages of simple preparation process, low cost, high specificity, high stability, short imaging period, low radiation dose, easiness in clinical conversion, etc.

## Description

### TECHNICAL FIELD

The present invention relates to the technical field of molecular imaging probe and, in particular, to a method for preparing a CD70-specific theranostic molecular imaging probe and use thereof.

### BACKGROUND TECHNOLOGY

In 1993, Belgian scientists Hamers et al. reported for the first time in Nature that a naturally occurring light chain-deficient antibody existed in alpaca peripheral blood (Nature. 1993;363(6428):446-8.). Such antibodies with special structural domains are known as heavy-chain antibodies (HCAbs). Molecular biological techniques can be used to clone the variable domain of a heavy-chain antibody, obtaining an antigen-binding fragment only including the heavy-chain variable domain, known as a nanobody (VHH, Variable domain of Heavy chain of Heavy-chain antibody). VHH crystals are 2.5 nm in diameter and 4 nm in length, and their molecular weight is only 15 KDa. This is why they are called nanobodies (Nanobodies^{®} is a registered trademark of Ablynx). To date, nanobodies are the smallest known antibodies capable of binding to target antigens. They have a variety of advantages, including high affinity, small molecular weights, inexpensive preparation (because they can be expressed both in *E. coli* and in eukaryotic expression systems such as yeasts and Chinese hamster ovary (CHO) cells), and easy clinical translation and generalization for application.

In recent years, nanobodies have been intensively studied for their potential use as targeting vectors for constructing molecular imaging probes (Theranostics. 2014;4(4):386-98.; J Nucl Med. 2022 Oct;63(10): 1705-1709.). To date, many nuclides with short half-lives have been used in labeling of nanobodies and preparation of nanobody molecular imaging probes. A technetium-99m (^{99m}Tc; T_{*1*/*2*} = 6.02 h) labeled nanobody probe targeting programmed death ligand 1 (PD-L1) has been successfully translated into clinical practice for non-invasive diagnosis of patients with non-small cell lung cancer (J Nucl Med. 2019;60(9):1213-1220.) A gallium-68 (⁶⁸Ga; T_{*1*/}*₂ =* 1.1 h) labeled nanobody probe targeting human epidermal growth factor receptor 2 (HER2) has also been successfully translated into clinical practice for non-invasive diagnosis of breast cancer (J Nucl Med. 2016;57(1):27-33.). These examples indicate that radionuclide-labeled nanobody probes are highly promising in clinical translation and use in non-invasive early diagnosis of human malignant tumors, visualization of critical pathogenic targets, screening of patients for monoclonal antibody (mAb) treatment and post- treatment mAb efficacy assessment.

Cluster of differentiation 70 (CD70), also known as CD27 ligand, is a type II transmembrane glycoprotein belonging to the tumor necrosis factor (TNF) superfamily. Binding of CD70 to its receptor CD27 induces priming of many signaling pathways and promotes gene transcription and cell proliferation and differentiation. Under normal conditions, CD70 is only transiently surface expressed on activated T and B cells and mature dendritic cells. In recent years, elevated expression of CD70 has been observed in a variety of malignant hematological and solid tumors in studies. Compared with normal renal tissue, significantly elevated CD70-expressing has been found in renal cell carcinoma (RCC), especially in the clear cell and sarcomatoid subtypes, and such higher CD70-expressing has been found to be associated with poor prognosis. CD70 expressed on tumor cells binds CD27 on the surface of T cells to activate the pro-apoptotic protein Siva, which triggers apoptosis of immune cells through cytotoxic mechanisms, leading to immune escape. Such differences in expression of CD70 in normal tissues and tumors make it a highly promising tumor-specific marker free of potential side effects. At present, there have been CD70-targeting drugs in clinical trials, such as mAbs, antibody-drug conjugates and chimeric antigen receptor T cells. The anti-CD70 mAb SGN-CD70A has been tested in patients with metastatic RCC in a phase I clinical trial, showing a clinical benefit rate of 78%. Therefore, there is an urgent need to develop a CD70-targeting diagnostic tool for visualizing and monitoring CD70-expressing in solid tumors. On the basis of such companion diagnostic tools, novel CD70-targeting therapies can also be developed.

The applicant's series of previous basic and clinical studies have shown that, sensibly combining the outstanding targeting specificity of antibodies with the superior sensitivity and resolution of positron emission tomography (PET), immuno-PET can better visualize the distribution and abundance of a target of interest, especially a heterogeneously expressed one, in the body and can better predict the response to targeted therapy or immunotherapy, compared to immunohistochemical (IHC) or other traditional predictive markers (Chem Rev.2020;120(8):3787-3851) . However, clinical translation and application of mAb-based immuno-PET imaging probes have been severely limited due to expensive preparation, large molecular weights, long *in vivo* circulation times, long imaging cycles, high radiation doses and significant toxic and side effects.

To date, there has been no CD70-specific molecular imaging probe or nuclide-labeled theranostic probe reported in clinical practice or literature. In order to fill this gap in the art, those skilled in the art seek to develop a nanobody-based immuno-PET imaging probe with low preparation cost, a small molecular weight, short *in vivo* circulation time, short imaging cycle, low radiation dose and ease of clinical translation and application.

### CONTENT OF THE INVENTION

In order to overcome the above-described problems, it is an object of the present invention to provide a method for preparing a CD70-specific theranostic molecular imaging probe and use thereof.

The above object is attained by each of the following aspects of the present invention.

In a first aspect, the present invention provides a CD70-specific nanobody, which is B3 or B6.

The B3 has an amino acid sequence as shown in SEQ ID NO. 1, and the B6 has an amino acid sequence as shown in SEQ ID NO. 3.

Preferably, the B3 has a gene sequence as shown in SEQ ID NO. 2, and the B6 has a gene sequence as shown in SEQ ID NO. 4.

In a second aspect, the present invention provides a use of the CD70-specific nanobody as defined above in the manufacture of a CD70-specific nanobody fusion protein.

The nanobody fusion protein may consist of a GGGGS linker of a variable amino acid length, a bridging albumin-binding domain (ABD) and the nanobody (B3 or B6). The GGGGS linker may contain 1-10 repeats. Specifically, it may have 1, 2, 3, 4, 5, 6, 7, 8, 9 or 10 repeats.

Preferably, the linker is a three-repeat linker having an amino acid sequence as shown in SEQ ID NO. 9 (i.e., GGGGSGGGGSGGGGS) and a gene sequence as shown in SEQ ID NO. 10.

In a third aspect, the present invention provides a CD70-specific nanobody fusion protein, which is ABDB3 or ABDB6.

The ABDB3 has an amino acid sequence as shown in SEQ ID NO. 5, and the ABDB6 has an amino acid sequence as shown in SEQ ID NO. 7.

Preferably, the ABDB3 has a gene sequence as shown in SEQ ID NO. 6, and the ABDB6 has a gene sequence as shown in SEQ ID NO. 8.

According to the present invention, the CD70-specific nanobody or CD70-specific nanobody fusion protein may be prepared according to a method including the steps of: cloning the gene sequence of the CD70-specific nanobody or CD70-specific nanobody fusion protein (as shown in SEQ ID NO. 2, 4, 6 or 8) into an expression vector; transforming the gene sequence into an expression host strain; culturing and expanding the transformed strain; inducing expression of the gene; purifying the expressed product, thereby obtaining the CD70-specific nanobody or CD70-specific nanobody fusion protein.

In a fourth aspect, the present invention provides use of the CD70-specific nanobody or CD70-specific nanobody fusion protein as defined above in the manufacture of a CD70-specific theranostic molecular imaging probe.

In a fifth aspect, the present invention provides a CD70-specific theranostic molecular imaging probe, which includes a tumor-targeting group and a radionuclide.

The tumor-targeting group is selected from the CD70-specific nanobodies or CD70-specific nanobody fusion proteins as defined above.

Preferably, the radionuclide is selected from ⁶⁸Ga, ¹⁸F, ⁶⁴Cu or ⁸⁹Zr, wherein when the radionuclide is selected from ⁶⁸Ga, ⁶⁴Cu or ⁸⁹Zr, the probe further includes a chelating agent selected from *p*-SCN-Bn-NOTA or*p*-SCN-Bn-Deferoxamine.

Preferably, the probe is any one of the followings: ⁶⁸Ga-labeled monovalent nanobody probes [⁶⁸Ga]Ga-NOTA-B3 and [⁶⁸Ga]Ga-NOTA-B6, ⁶⁸Ga-labeled nanobody fusion protein probes [⁶⁸Ga]Ga-NOTA-ABDB3 and [⁶⁸Ga]Ga-NOTA-ABDB6, ¹⁸F-labeled monovalent nanobody probes [¹⁸F]F-B3 and [¹⁸F]F-B6, and ⁸⁹Zr-labeled nanobody fusion protein probes [⁸⁹Zr]Zr-DFO-ABDB3 and [⁸⁹Zr]Zr-DFO-ABDB6.

Preferably, when the probe includes a tumor-targeting gene, the chelating agent and the radionuclide ⁶⁸Ga, ⁶⁴Cu or ⁸⁹Zr, the probe is prepared according to a method including the steps of:
modifying the tumor-targeting gene with the chelating agent, obtaining a modified nanobody; and then labeling the modified nanobody with the radionuclide ⁶⁸Ga or ⁸⁹Zr, thereby obtaining the probe.

Preferably, when the probe includes a tumor-targeting gene and the radionuclide ¹⁸F, the probe is prepared according to a method including the steps of:
labeling a precursor with the radionuclide ¹⁸F, which is a small-molecule compound, obtaining a ¹⁸F-labeled precursor;
preparing a nanobody randomly conjugated with DBCO; and
obtaining the probe from a click chemistry reaction between the ¹⁸F-labeled precursor and the nanobody randomly conjugated with DBCO.

Compared with the prior art, the present invention has the advantageous effects as described below.

The present invention has realized non-invasive visualization of the expression of human CD70 molecules, enabling non-invasive diagnosis of renal cell carcinoma. The probes of the present invention have advantages including but not limited to simple preparation, low cost, high specificity, high stability, a short imaging cycle, low radiation dose, easy clinical translation.

### DESCRIPTION OF THE DRAWINGS

Other features, objects and advantages of the present invention will become more apparent upon a reading of the following detailed description of non-limiting embodiments thereof when taken in conjunction with the accompanying drawings, in which:
FIG. 1 shows the expression of nanobodies B3 and B6 determined using SDS-PAGE;
FIG. 2 shows the expression of nanobody fusion proteins ABDB3 and ABDB6 determined using SDS-PAGE;
FIG. 3 shows CD70 staining results of renal cell carcinoma (RCC) patient-derived xenograft (PDX) models, No. 62 PDX.
FIG. 4 shows affinity measurements of nanobodies B3 and B6 and nanobody fusion proteins ABDB3 and ABDB6 to human CD70, in which the curves in each series correspond to concentrations increasing from the bottom upwards.
FIG. 5 shows affinity measurements of nanobodies B3 and B6 and nanobody fusion proteins ABDB3 and ABDB6 to human and murine serum albumins, in which the curves in each series correspond to concentrations increasing from the bottom upwards.
FIG. 6 shows radiochemical purity measurements of probes [⁶⁸Ga]Ga-NOTA-B3, [⁶⁸Ga]Ga-NOTA-B6, [⁶⁸Ga]Ga-NOTA-ABDB3 and [⁶⁸Ga]Ga-NOTA-ABDB6 obtained by a radio thin-layer chromatography (Radio-TLC) instrument.
FIG. 7 shows results of tests on diagnosing RCC based on immuno-PET imaging using [⁶⁸Ga]Ga-NOTA-B3: (A) PET/CT images; (B) region of interest (ROI) uptake; and (C) in vitro biodistribution data.
FIG. 8 shows results of tests on diagnosing RCC based on immuno-PET imaging using [⁶⁸Ga]Ga-NOTA-B6: (A) PET/CT images; (B) ROI uptake; and (C) in vitro biodistribution data.
FIG. 9 shows PET/CT images captured in blocking tests on RCC diagnosis based on immuno-PET imaging using [⁶⁸Ga]Ga-NOTA-B6.
FIG. 10 shows ROI uptake data in blocking tests on RCC diagnosis based on immuno-PET imaging using [⁶⁸Ga]Ga-NOTA-B6.
FIG. 11 shows in vitro biodistribution data of blocking tests on RCC diagnosis based on immuno-PET imaging using [⁶⁸Ga]Ga-NOTA-B6.
FIG. 12 shows PET/CT images in RCC diagnosis based on immuno-PET imaging using [⁶⁸Ga]Ga-NOTA-ABDB3 and [⁶⁸Ga]Ga-NOTA-ABDB6.
FIG. 13 shows ROI uptake data in RCC diagnosis based on immuno-PET imaging using [⁶⁸Ga]Ga-NOTA-ABDB3 and [⁶⁸Ga]Ga-NOTA-ABDB6.
FIG. 14 shows in vitro biodistribution data in RCC diagnosis based on immuno-PET imaging using [⁶⁸Ga]Ga-NOTA-ABDB3 and [⁶⁸Ga]Ga-NOTA-ABDB6.
FIG. 15 shows a comparison of ROI uptake data and in vitro biodistribution data derived from PET/CT images of RCC models between [⁶⁸Ga]Ga-NOTA-B3 and [⁶⁸Ga]Ga-NOTA-ABDB3.
FIG. 16 shows a comparison of ROI uptake data and in vitro biodistribution data derived from PET/CT images of RCC models between [⁶⁸Ga]Ga-NOTA-B6 and [⁶⁸Ga]Ga-NOTA-ABDB6.
FIG. 17 shows radiochemical purity measurements of probes [⁸⁹Zr]Zr-DFO-ABDB3 and [⁸⁹Zr]Zr-DFO-ABDB6 obtained by a Radio-TLC instrument.
FIG. 18 shows PET/CT images in RCC diagnosis based on immuno-PET imaging using [⁸⁹Zr]Zr-DFO-ABDB3.
FIG. 19 shows PET/CT images in RCC diagnosis based on immuno-PET imaging using [⁸⁹Zr]Zr-DFO-ABDB6.
FIG. 20 shows ROI uptake data in RCC diagnosis based on immuno-PET imaging using [⁸⁹Zr]Zr-DFO-ABDB3.
FIG. 21 shows ROI uptake data in RCC diagnosis based on immuno-PET imaging using [⁸⁹Zr]Zr-DFO-ABDB6.
FIG. 22 shows in vitro biodistribution data in RCC diagnosis based on immuno-PET imaging using [⁸⁹Zr]Zr-DFO-ABDB3 and [⁸⁹Zr]Zr-DFO-ABDB6.
FIG. 23 shows IHC staining of a tumor with a CD70-specific antibody E3Q1A.
FIG. 24 shows PET/CT images in RCC diagnosis based on immuno-PET imaging using [¹⁸F]F-B6.
FIG. 25 shows ROI uptake data and in vitro biodistribution data in RCC diagnosis based on immuno-PET imaging using [¹⁸F]F-B6.

### SPECIFIC IMPLEMENTATIONS

For a better understanding of the present invention, reference is made to the following detailed description of particular embodiments thereof, taken in conjunction with the accompanying drawings. It should be noted that the invention is not limited to the particular methods, protocols, cell lines, constructs, and reagents described herein, as such may vary. Unless otherwise indicated, all technical and scientific terms used herein have the same meaning as they would to one skilled in the art of the present invention. The terminology used herein is for purposes of describing particular embodiments only, and is not intended to limit the present invention.

The use of radiolabeled monoclonal antibodies has been largely hindered by their high costs, necessity of using long half-life radionuclides, cumbersome imaging processes within a cycle and related radiation exposure. In order to improve the clinical application of antibody-based diagnosis, the field of molecular imaging is actively exploring pre-targeted imaging strategies or the use of antibody derivatives with relatively small molecular weights for same-day imaging. Among small antibody forms, Camelidae-derived nanobodies or single-domain antibodies with a molecular weight of about 15 kDa are the smallest antigen-binding fragments. Small size, high affinity and ease of engineering make nanobodies an excellent alternative for molecular imaging (J Nucl Med. 2022 Oct;63(10):1705-1709.) In recent years, the applicant has focused on the development of nanobody-derived tracers and clinical translation thereof, which can give full play to their superior molecular imaging properties. Accordingly, the present application develops CD70-specific nanobodies B3 and B6 and use thereof in CD70-specific theranostic molecular imaging probes, obtaining radiolabeled monovalent nanobody probes (e.g., [⁶⁸Ga]Ga-NOTA-B3, [⁶⁸Ga]Ga-NOTA-B6 and [¹⁸F]F-B3 and [¹⁸F]F-B6).

Although radiolabeled monovalent nanobodies are ideal companion diagnostic tools, still further improvement would be desirable as their half-lives in vivo are too short and renal uptake is unfavorably high. In order to develop a theranostic platform, the applicant has also introduced an albumin-binding domain (ABD) targeting human/murine albumin into the monovalent nanobodies, obtaining CD70-specific nanobody fusion proteins (ABDB3 and ABDB6). These have been used for obtaining CD70-specific theranostic molecular imaging probes as radiolabeled nanobody fusion protein probes (e.g., [⁶⁸Ga]Ga-NOTA-ABDB3, [⁶⁸Ga]Ga-NOTA-ABDB6, [⁸⁹Zr]Zr-DFO-ABDB3, [⁸⁹Zr]Zr-DFO-ABDB6, [¹⁸F]F-ABDB3 and [¹⁸F]F-ABDB6). In this way, the half-lives of the monovalent nanoantibody derivatives in in living organisms are significantly extended, further optimizing the pharmacokinetics and pharmacodynamics of the molecular imaging probes. Studies have shown that bispecific nanobody derivatives that target both tumor antigens and albumin improve in vivo biodistribution and can be used as carriers for the development of theranostic toolboxes. CD70-specific theranostic molecular imaging probes constructed in accordance with the present invention can non-invasively visualize CD70-expressing in tumors and provide a better method for the diagnosis and monitoring of CD70-positive solid tumors.

### Example 1

In this example, CD70-specific nanobodies B3 and B6 were prepared according to a method of the present invention. The CD70-specific nanobody B3 had an amino acid sequence as shown in SEQ ID NO. 1 and a base sequence as shown in SEQ ID NO. 2. The CD70-specific nanobody B6 had an amino acid sequence as shown in SEQ ID NO. 3 and a base sequence as shown in SEQ ID NO. 4.

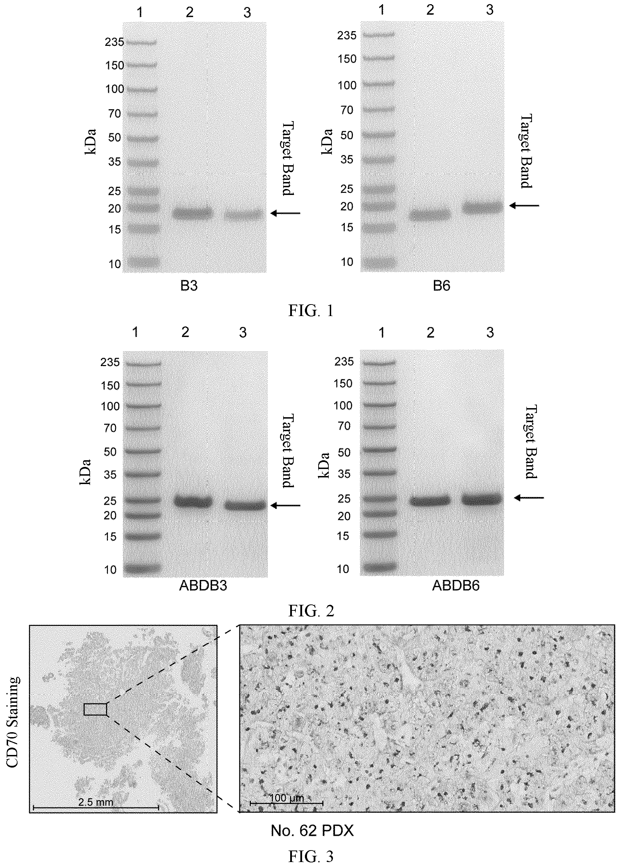

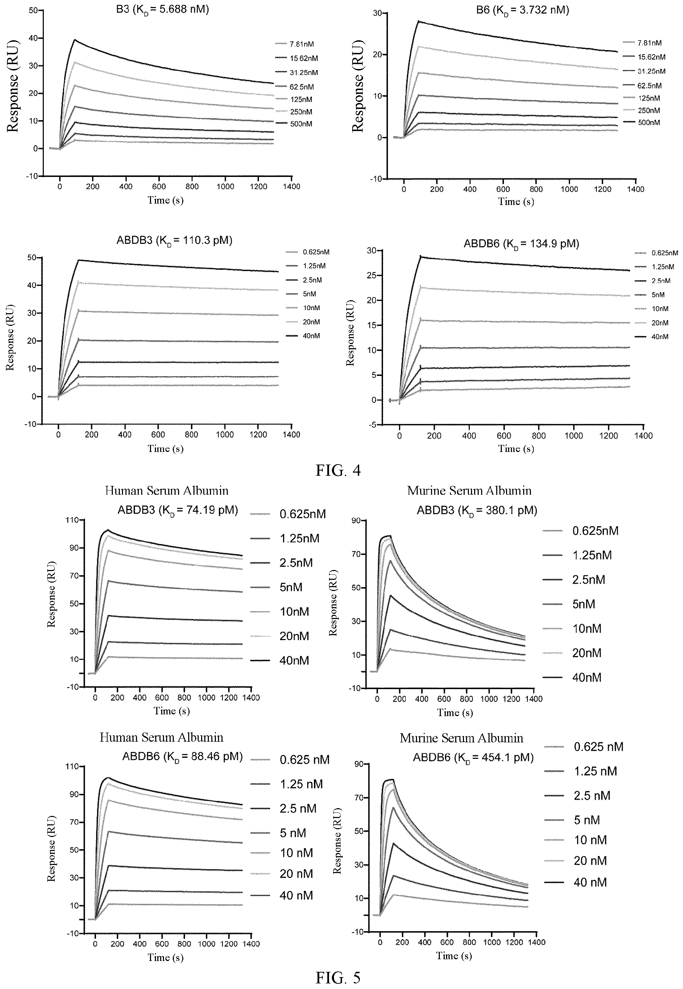

The method included the steps as described below.
1) For each of SEQ ID NOs. 2 and 4, a conventional molecular biological technique was employed to clone the base sequence into a pET-30a(+) expression vector, obtaining a plasmid DNA containing the target antibody (B3 or B6).
2) The target antibodies were expressed in *Escherichia coli* (*E. coli*).
   *2.1 Transformation into E. coli.* BL21 (DE3) competent cells stored at -80 °C were taken out and thawed on ice. The plasmid DNAs containing the target antibodies were separately added, 100 mg each, to the BL21 (DE3) competent cells, followed by gentle mixing. The competent cells were incubated on ice for 30 minutes, subjected to a heat shock in a quiescent state at 42 °C for 90 seconds, kept on ice for 3 minutes, added with 100µl of LB medium at ambient temperature, incubated for 60 minutes at 200 rpm and 37 °C and plated on LB agar plate containing 50 µg/ml of kanamycin. The agar plate was inverted and incubated at 37 °C overnight.
   *2.2 Small-Scale Expression Test.* Well-dispersed monoclonal colonies were randomly picked from the agar plate and separately inoculated and incubated in LB medium containing 50 µg/ml of kanamycin. The incubation was conducted at 200 rpm and 37 °C. When OD600 reached 0.6-0.8 as measured, isopropyl β-D-1-thiogalactopyranoside (IPTG) was added to the cultures to a concentration of 0.5 mM, which were then separately incubated at 15 °C for 16 hours, or at 37 °C for 4 hours (both these incubation conditions were feasible).
   *2.3 Determination of Protein Expression Using SDS-PAGE (Sodium Dodecyl Sulfate-Polyacrylamide Gel Electrophoresis*). 450µL of each incubated culture was taken and centrifuged, and the cell pellet was collected. The cells were disrupted and lysed using ultrasound waves for 1 minute in 300 µl of a lysis solution (50 mM Tris; 150 mM NaCl; 5% glycerol; pH=8.0). Each lysed cell sample was heated at 100 °C for 10 minutes, centrifuged at 15,000 rpm for 5 minutes and tested. Expression of the target antibodies B3 and B6 is shown in FIG. 1, in which Lane 1 represents a Western blot marker; Lane 2, reduced B3 or B6; and Lane 3, unreduced B3 or B6.
3) Affinity of the target antibodies B3 and B6 to human CD70 was tested. Each of the monovalent nanobodies (B3 or B6) was loaded as a mobile phase at various concentrations on Biacore chips immobilized thereon with an extracellular domain of human recombinant CD70 and then eluted. The test results are shown in FIG. 4. KD values of B3 and B6, which represented their affinity, were 5.688 nM and 3.732 nM, respectively.

### Example 2

In this example, CD70-specific nanobody fusion proteins ABDB3 and ABDB6 were prepared according to a method of the present invention, each consisting of a GGGGS linker of a variable amino acid length, a bridging albumin-binding domain (ABD) and a nanobody (B3 or B6). The GGGGS linker could have 1, 2, 3, 4, 5, 6, 7, 8, 9 or 10 repeats. The nanobody fusion proteins ABDB3 and ABDB6 prepared in this example each contained a three-repeat linker having an amino acid sequence as shown in SEQ ID NO. 9 and a gene sequence as shown in SEQ ID NO. 10.

The CD70-specific nanobody fusion protein ABDB3 had an amino acid sequence as shown in SEQ ID NO. 5 and a base sequence as shown in SEQ ID NO. 6. The CD70-specific nanobody fusion protein ABDB6 had an amino acid sequence as shown in SEQ ID NO. 7 and a base sequence as shown in SEQ ID NO. 8.

The method included the steps as described below.
1) For each of SEQ ID NO. 6 and 8, a conventional molecular biological technique was used to clone the base sequence into a pET-30a(+) expression vector, obtaining a plasmid DNA coding the target fusion protein (ABDB3 or ABDB6).
2) The target fusion proteins were expressed in *Escherichia coli* (*E. coli*).
   The same operations were performed as in step 2) of Example 1. Expression of the target fusion proteins ABDB3 and ABDB6 is shown in FIG. 2, in which Lane 1 represents a Western blot marker; Lane 2, reduced ABDB3 or ABDB6; and Lane 3, unreduced ABDB3 or ABDB6.
3) Affinity of the target fusion proteins ABDB3 and ABDB6 to human CD70 was tested. Each of the monovalent nanobodies (ABDB3 and ABDB6) was loaded as a mobile phase at various concentrations on Biacore chips immobilized thereon with an extracellular domain of human recombinant CD70 and then eluted. The test results are shown in FIG. 4. KD values of ABDB3 and ABDB6 were 110.3 pM and 134.9 pM, respectively.
4) Affinity of the target fusion proteins ABDB3 and ABDB6 to human and murine serum albumins was tested. Each of ABDB3 and ABDB6 was loaded as a mobile phase at concentrations in a gradient on Biacore chips immobilized thereon separately with recombinant human and murine serum albumins and then eluted. The test results are shown in FIG. 5. KD values of ABDB3 for human and murine serum albumins were 74.19 pM and 380.1 pM, respectively. KD values of ABDB6 for human and murine serum albumins were 88.46 pM and 454.1 pM, respectively.

### Example 3

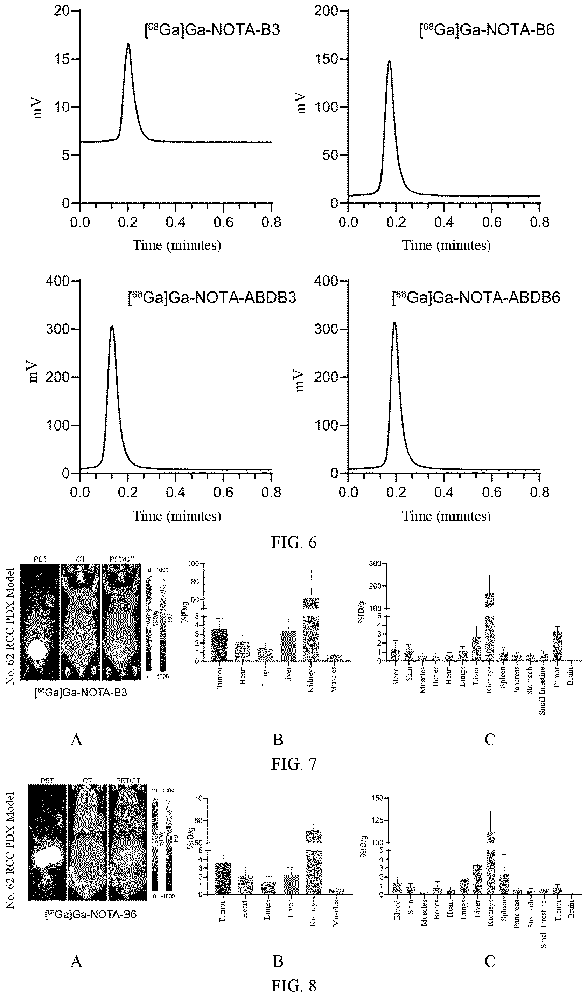

In this example, human CD70-specific ⁶⁸Ga-labeled monovalent nanobody probes [⁶⁸Ga]Ga-NOTA-B3 and [⁶⁸Ga]Ga-NOTA-B6 and human CD70-specific ⁶⁸Ga-labeled nanobody fusion protein probes [⁶⁸Ga]Ga-NOTA-ABDB3 and [⁶⁸Ga]Ga-NOTA-ABDB6 were prepared according to a method of the present invention, which included the steps as described below.
1) Intermediates NOTA-B3, NOTA-B6, NOTA-ABDB3 and NOTA-ABDB6 were prepared by modifying B3, B6, ABDB3 and ABDB6 with *p*-SCN-Bn-NOTA. 1 mg of B3, B6, ABDB3 or ABDB6 was dissolved in 1 mL of phosphate-buffered saline (PBS), and the resulting nanobody solution was adjusted to a pH of 9.0-10 with 0.1 mL of a 0.1 M sodium carbonate (Na₂CO₃) buffer (pH=11.4). The volume of the reaction system was 1.1 mL. *p*-SCN-Bn-NOTA (CAS No. 170597-66-8; Macrocyclics) freshly dissolved in dimethyl sulfoxide (DMSO) was added to the nanobody solution at a molar ratio of *p*-SCN-Bn-NOTA to the nanobody or fusion protein of 10: 1, and the resulting reaction system was kept at room temperature for 2 hours to allow the reaction to proceed. The NOTA-modified nanobody, i.e., NOTA-B3, NOTA-B6, NOTA-ABDB3 or NOTA-ABDB6, was purified using a pre-equilibrated PD-10 desalting column (GE Healthcare) with PBS as a mobile phase. After being collected, NOTA-B3, NOTA-B6, NOTA-ABDB3 or NOTA-ABDB6 was concentrated using an ultra-centrifugal filter (Merck Millipore) with a cutoff of 10 KDa and then measured for concentration on NanoDrop, followed by storage in aliquots at -20 °C for subsequent use.
2) [⁶⁸Ga]Ga-NOTA-B3, [⁶⁸Ga]Ga-NOTA-B6, [⁶⁸Ga]Ga-NOTA-ABDB3 and [⁶⁸Ga]Ga-NOTA-ABDB6 were prepared from ⁶⁸Ga-labeled NOTA-B3, NOTA-B6, NOTA-ABDB3 and NOTA-ABDB6. A germanium-gallium generator (Eckert & Ziegler Radiopharma Inc.) was eluted with 4 mL of a 0.05 M hydrochloric acid (HCl) solution, and equal volumes of the ⁶⁸Ga-containing eluate with an activity of about 370-555 MBq were collected. Intermediate fractions of the ⁶⁸Ga-containing eluate with the highest activity, amounting to 2 mL, were taken, and 0.1 mL of a 1 M sodium acetate (NaoAc) solution was added thereto, adjusting its pH to 4.0-4.5. 200 µg of the stored conjugate NOTA-B3, NOTA-B6, NOTA-ABDB3 or NOTA-ABDB6 was added to the ⁶⁸Ga-containing eluate, and the resulting reaction system, with a volume<2.5 mL, was kept in a thermostatic shaker at room temperature for 5-10 minutes to allow the reaction to proceed. After the labeling reaction, free ⁶⁸Ga was removed again by elution on a pre-equilibrated PD-10 desalting column using PBS as a mobile phase. The purified final product was obtained at a non-decay corrected radiochemical yield (RCY) >50%.
3) Quality control of [⁶⁸Ga]Ga-NOTA-B3, [⁶⁸Ga]Ga-NOTA-B6, [⁶⁸Ga]Ga-NOTA-ABDB3 and [⁶⁸Ga]Ga-NOTA-ABDB6 was verified. 10-µL samples of the [⁶⁸Ga]Ga-NOTA-B3, [⁶⁸Ga]Ga-NOTA-B6, [⁶⁸Ga]Ga-NOTA-ABDB3 and [⁶⁸Ga]Ga-NOTA-ABDB6 probes prepared in step 2) were loaded on silica gel plates and measured for radiochemical purity (RCP) on a radio thin-layer chromatography (Radio-TLC) instrument (Eckert & Ziegler Radiopharma Inc.) using a 0.1 M sodium citrate solution (pH=5) as a mobile phase. As shown in FIG. 6, [⁶⁸Ga]Ga-NOTA-B3, [⁶⁸Ga]Ga-NOTA-B6, [⁶⁸Ga]Ga-NOTA-ABDB3 and [⁶⁸Ga]Ga-NOTA-ABDB6, which were freshly prepared, all exhibited RCP of more than 99%.

### Example 4

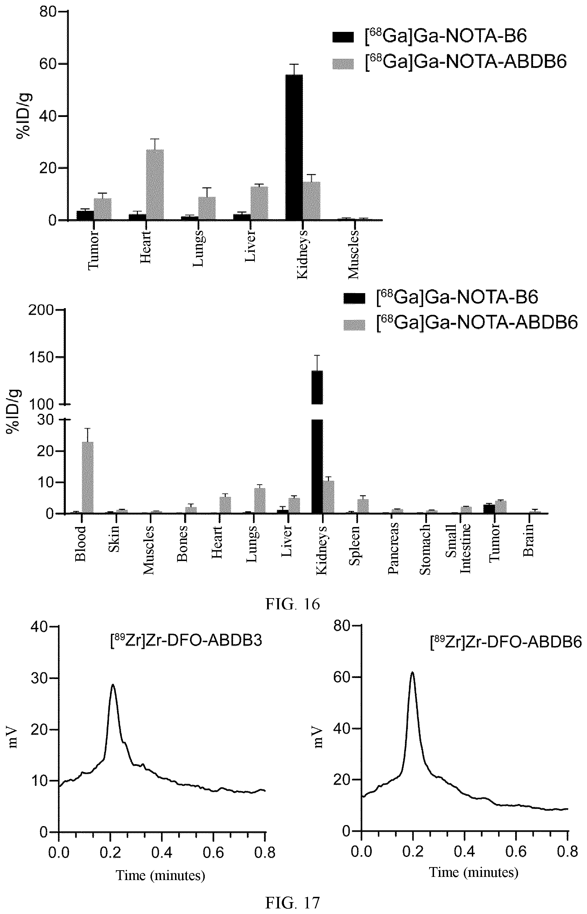

In this example, human CD70-specific ⁸⁹Zr-labeled nanobody fusion protein probes [⁸⁹Zr] Zr-DFO-ABDB3 and [⁸⁹Zr] Zr-DFO-ABDB6 were prepared according to a method of the present invention, which included the steps as described below.
1) Intermediates DFO-ABDB3 and DFO-ABDB6 were prepared by modifying ABDB3 and ABDB6 with *p*-SCN-Bn-Deferoxamine. 3 mg of ABDB3 or ABDB6 was dissolved in 1 mL of phosphate-buffered saline (PBS), and the resulting nanobody solution was adjusted to a pH of 8.9-9.1 with 0.1 mL of a 0.1 M sodium carbonate (Na₂CO₃) buffer (pH=11.4). The volume of the reaction system was 1.1 mL. DFO (CAS No. 170597-66-8; Macrocyclics) freshly dissolved in dimethyl sulfoxide (DMSO) was added to the nanobody solution at a molar ratio of DFO to ABDB3 or ABDB6 of 5: 1, and the resulting reaction system was kept at room temperature for 30 minutes to allow the reaction to proceed. The DFO-modified nanobody, i.e., DFO-ABDB3 or DFO-ABDB6 was purified using a pre-equilibrated PD-10 desalting column (GE Healthcare) with PBS as a mobile phase. After being collected, DFO-ABDB3 or DFO-ABDB6 was concentrated using an ultra-centrifugal filter (Merck Millipore) with a cutoff of 10 KDa and then measured for concentration on NanoDrop, followed by storage in aliquots at -20 °C for subsequent use.
2) [⁸⁹Zr]Zr-DFO-ABDB3 and [⁸⁹Zr]Zr-DFO-ABDB6 were prepared from ⁸⁹Zr-labeled DFO-ABDB3 and DFO-ABDB6. 450 µl of a 100-MBq ⁸⁹Zr-oxalate solution was prepared and adjusted with a 1 M Na₂CO₃ buffer solution to pH=7. After that, 500 µl of a 0.5 M HEPES solution (pH=7.1-7.3) and 200 µg of DFO-ABDB3 or DFO-ABDB6 were added stepwise to the reaction solution, and the resulting reaction system was kept in a thermostatic shaker at room temperature for 1 hour to allow the reaction to proceed. After the labeling reaction, free ⁸⁹Zr was removed again by elution on a pre-equilibrated PD-10 desalting column using PBS as a mobile phase, obtaining the purified final product [⁸⁹Zr]Zr-DFO-ABDB3 or [⁸⁹Zr]Zr-DFO-ABDB6.
3) Quality control of [⁸⁹Zr]Zr-DFO-ABDB3 and [⁸⁹Zr]Zr-DFO-ABDB6 was verified. 10-µL samples of the [⁸⁹Zr]Zr-DFO-ABDB3 and [⁸⁹Zr]Zr-DFO-ABDB6 probes prepared in step 2) were loaded on silica gel plates and measured for RCP on a Radio-TLC instrument (Eckert & Ziegler Radiopharma Inc.) using a 0.1 M sodium citrate solution (pH=5) as a mobile phase. As shown in FIG. 17, [⁸⁹Zr]Zr-DFO-ABDB3 and [⁸⁹Zr]Zr-DFO-ABDB6, which were freshly prepared, both exhibited RCP of more than 90%.

### Example 5

In this example, human CD70-specific ¹⁸F-labeled monovalent nanobody probes [¹⁸F]F-B3 and [¹⁸F]F-B6 were prepared according to a method of the present invention, which included the steps as described below.
1) ¹⁸F[F]-RJDJ01 was prepared according to the method described in Example 2 of Pat. App. Pub. No. CN113476619A.
2) DBCO-B3 and DBCO-B6 were prepared. 1 mg of B3 or B6 was dissolved in phosphate-buffered saline (PBS) to a final volume of about 1 mL, and 80-100 µL of a 0.1 M sodium carbonate (Na₂CO₃) buffer solution was added to the nanobody solution to adjust its pH to 9.0-10. DBCO-NHS ester (CAS No. 1353016-71-3; MeloPEG) freshly dissolved in dimethyl sulfoxide (DMSO) was added to the nanobody solution at a molar ratio of DBCO-NHS ester to B3/B6 of 10: 1, and the resulting reaction system was kept at room temperature for 2 hours to allow the reaction to proceed. The nanobody randomly conjugated with DBCO, i.e., DBCO-B3 or DBCO-B6, was purified using a pre-equilibrated PD-10 desalting column (GE Healthcare) with PBS as a mobile phase. After being collected, the nanobody DBCO-B3 or DBCO-B6 was concentrated using an ultra-centrifugal filter (Merck Millipore) with a cutoff of 10 KDa and measured for concentration on NanoDrop, followed by storage in a refrigerator at 4 °C for subsequent use.
3) [¹⁸F]F-B3 and [¹⁸F]F-B6 were prepared from a click chemistry reaction. 332 µL(320 µg) of the DBCO-B3 or DBCO-B6 nanobody solution was taken, and 20 mL of ¹⁸F[F]- RJDJ01 was added thereto. The resulting reaction system was kept in a thermostatic shaker at 45 °C for 45 minutes to allow the reaction to proceed. After that, unreacted 18F-RJDJ01 was removed again using a pre-equilibrated PD-10 desalting column with PBS as a mobile phase, and the purified final product [¹⁸F]F-B3 or [¹⁸F]F-B6 was collected.

Additionally, [¹⁸F]F-ABDB3 and [¹⁸F]F-ABDB6 could be prepared as final products according to the same method as described above.

### Validation Examples

### 1) Establishment of Tumor-Bearing Murine Models Positive in the Expression of CD70

Renal cell carcinoma (RCC) patient-derived xenograft (PDX) models, No. 62 PDX, positive in expression of CD70 were identified by immunohistochemical (IHC) staining using an antihuman CD70 monoclonal antibody (E3Q1A, 69209, Cell Signaling Technology) as a primary antibody, as shown in FIG. 3. RCC PDX models were established by subcutaneously inoculating 2mm×2mm×2mm No. 62 PDX tissue chunks into right shoulder of NCG (NOD-Prkdcem26Cd52Il2rgem26Cd22/Nju) mice.

### 2) RCC Diagnosis Tests Based on Immuno-PET Imaging Using [⁶⁸Ga]Ga-NOTA-B3 and [⁶⁸Ga]Ga-NOTA-B6

PET/CT images of small animals involved in these tests were all captured using an IRIS small-animal PET/CT scanner (Inviscan Imaging Systems). Each murine model (three in each group) was injected with 3.7-7.4 MBq of [⁶⁸Ga]Ga-NOTA-B3 or [⁶⁸Ga]Ga-NOTA-B6 via the tail vein. One hour after the injection, the mice were anesthetized with isoflurane mixed in oxygen (at a concentration of 2%). After being deeply anesthetized, the mice were placed supine on the PET/CT scanning bed, and PET and CT images were captured sequentially. Image reconstruction was done using software included in the IRIS system, as shown in FIG. 7 and FIG. 8. The OsiriX Lite imaging workstation (Pixmeo SARL) was used to delineate regions of interest (ROIs) corresponding to the heart and other major tissues and organs (liver, lungs, kidneys and muscles) on the reconstructed PET images and estimate radiotracer uptake values of the major tissues and organs in percent of injected dose per gram (%ID/g). The PET/CT images are shown in FIG. 7A and FIG. 8A, ROI uptake in FIG. 7B and FIG. 8B, and in vitro biodistribution data in FIG. 7C and FIG. 8C. Analysis of in vivo distribution of [⁶⁸Ga]Ga-NOTA-B3 and [⁶⁸Ga]Ga-NOTA-B6 in the delineated ROIs revealed high uptake of the CD70-specific nanobody probes [⁶⁸Ga]Ga-NOTA-B3 and [⁶⁸Ga]Ga-NOTA-B6 in the tumor tissue, and high nonspecific uptake in the major excretory (kidneys) and metabolic (liver) organs. The in vitro biodistribution results further revealed the in vivo distribution of the probes in the major tissues and organs. No significant difference was found in effectiveness of [⁶⁸Ga]Ga-NOTA-B3 and [⁶⁸Ga]Ga-NOTA-B6 on RCC diagnosis in the models according to analysis of the ROI uptake and biodistribution data of the two probes in the tumor models. These results suggested that the [⁶⁸Ga]Ga-NOTA-B3 and [⁶⁸Ga]Ga-NOTA-B6 probes enabled non-invasive visualization of CD70 expression.

### 3) Blocking Tests on RCC Diagnosis Based on Immuno-PET Imaging Using [⁶⁸Ga]Ga-NOTA-B6

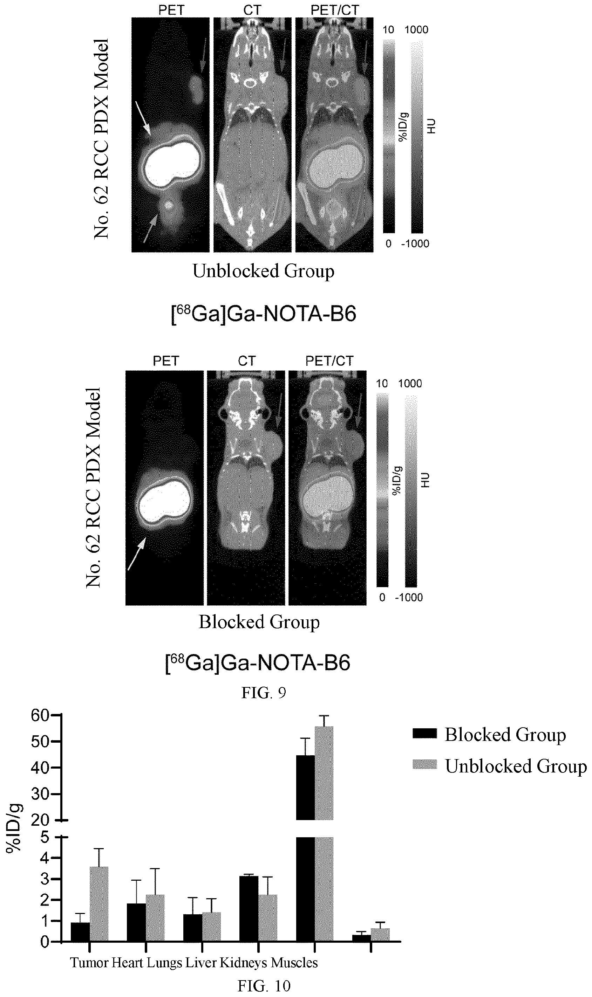

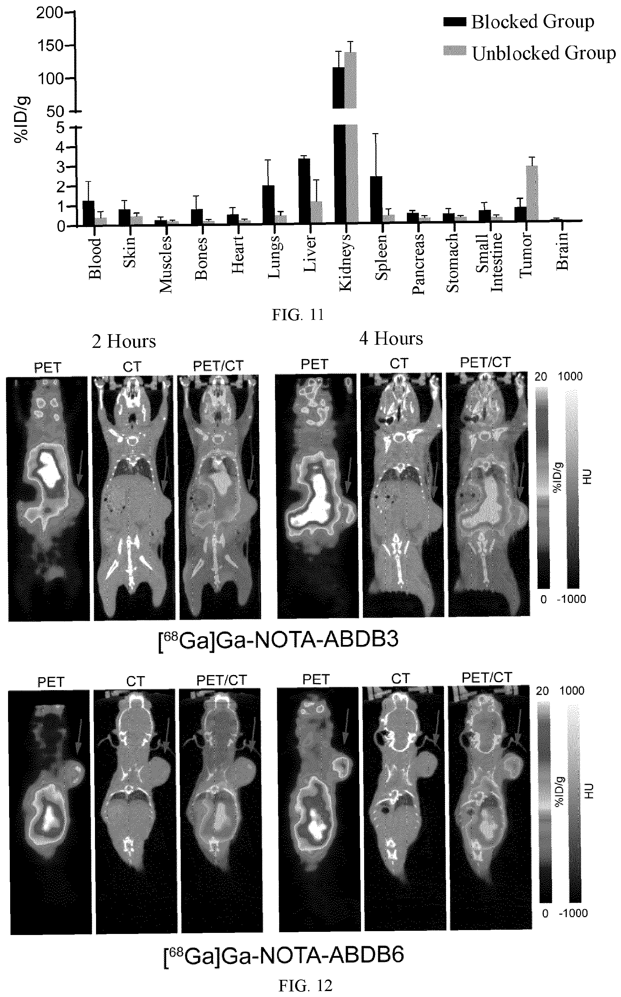

A test group was injected with ABDB6 (20 mg/kg), but an unblocked (control) group was not, 48 hours before both receiving an injection of the imaging agent ([⁶⁸Ga]Ga-NOTA-B6). PET images of both groups were captured 1 hour after the injection of the imaging agent. The PET/CT results are presented in FIG. 9, in which the top and bottom details show images of the unblocked and blocked groups, respectively, which indicated that blocking CD70 on the surface of tumor cells with ABDB6 could significantly reduce the uptake of the monovalent nanobody probe in tumor tissue. According to statistical analysis of ROI uptake data and in vitro biodistribution data, the tumor uptake of the blocked group is considerably lower than that of the unblocked group, as shown in FIG. 10 and FIG. 11.

### 4) RCC Diagnosis Tests Based on Immuno-PET Imaging Using [⁶⁸Ga]Ga-NOTA-ABDB3 and [⁶⁸Ga]Ga-NOTA-ABDB6

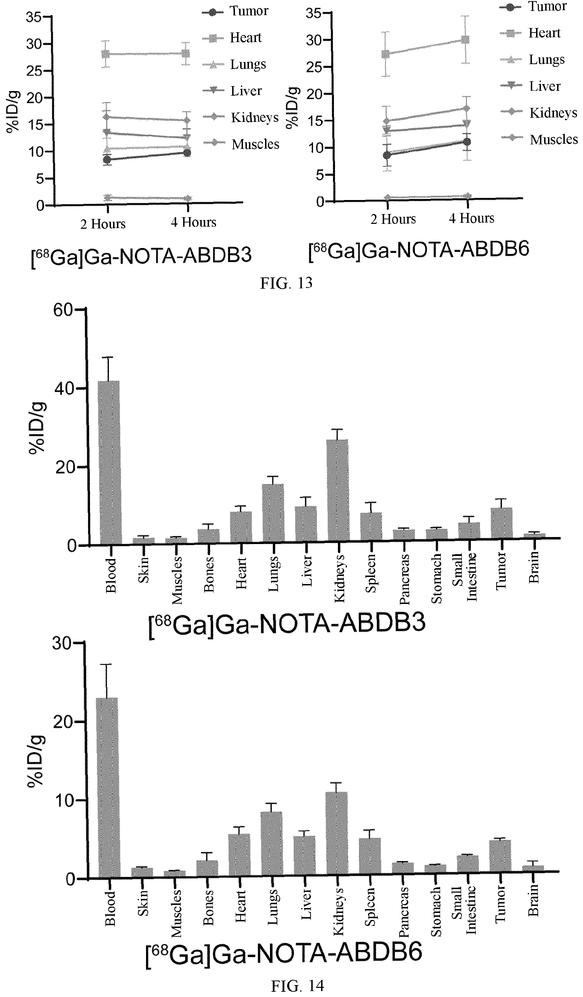

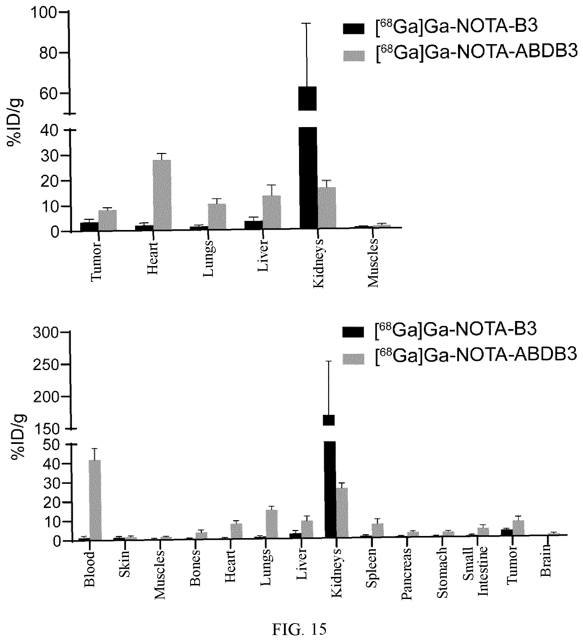

The same operations as in step 2) above were performed, and the test results are shown in FIG. 12. PET/CT images of RCC models (No. 62 PDX) injected with [⁶⁸Ga]Ga-NOTA-ABDB3 and [⁶⁸Ga]Ga-NOTA-ABDB6 captured at multiple points of time (2 and 4 hours after injection) still showed high uptake of the CD70-specific nanobody fusion protein probes [⁶⁸Ga]Ga-NOTA-ABDB3 and [⁶⁸Ga]Ga-NOTA-ABDB6 in tumor tissue. As revealed by the ROI uptake data of FIG. 13, uptake in the tumor increased over time within 4 hours. The in vitro distribution data of FIG. 14 further confirmed the enrichment of the probes in the tumor. A comparison of the ROI uptake data and in vitro biodistribution data derived from the PET/CT images of the RCC models between the two types of probes, i.e., [⁶⁸Ga]Ga-NOTA-B3 v.s. [⁶⁸Ga]Ga-NOTA-ABDB3 and [⁶⁸Ga]Ga-NOTA-B6 v.s. [⁶⁸Ga]Ga-NOTA-ABDB6, as shown in FIG. 15 and FIG. 16, additionally revealed that the nanobody fusion protein probes did not degrade in the ability to non-invasively visualize CD70 expression in the tumor and showed far less uptake in the kidney.

### 5) RCC Diagnosis Tests Based on Immuno-PET Imaging Using [⁸⁹Zr]Zr-DFO-ABDB3 and [⁸⁹Zr]Zr-DFO-ABDB6

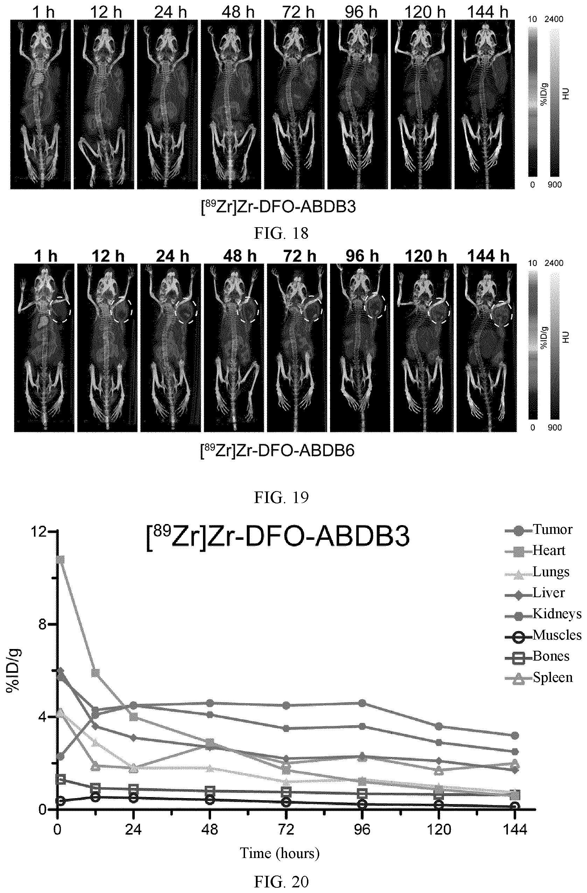

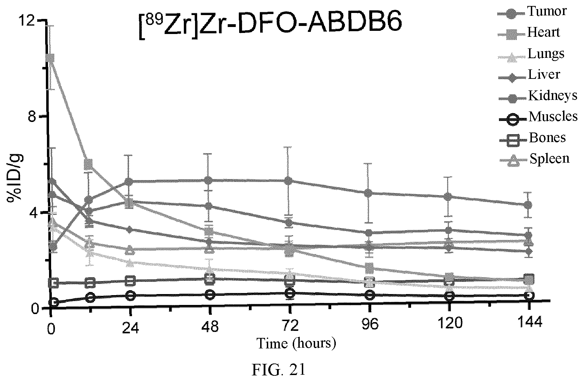

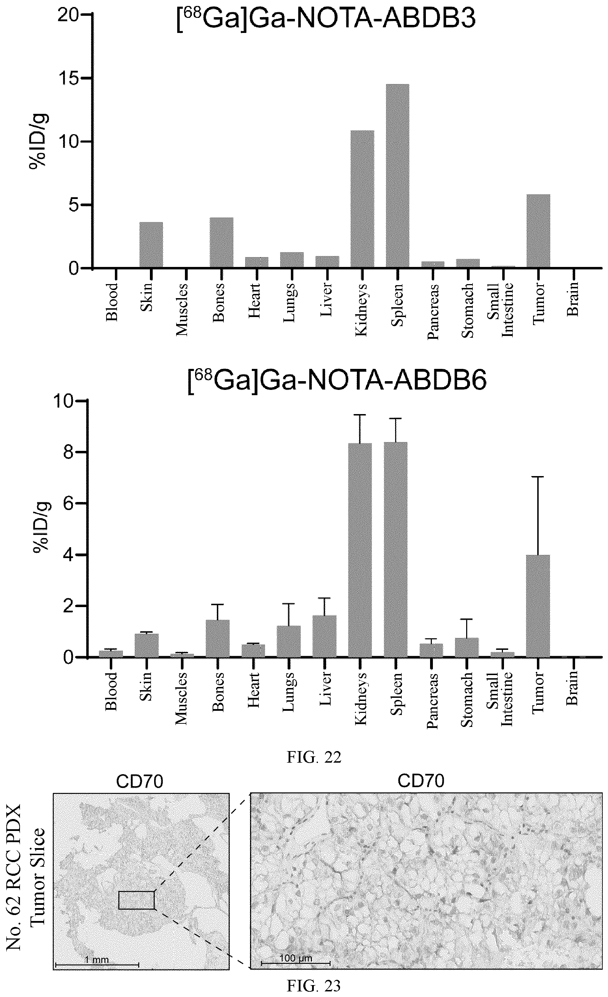

The same operations as in step 2) above were performed. PET/CT images were captured 1 , 12 , 24,48,72 , 96 , 120 and 144 hours after injection of the probes, as shown in FIG. 18 and FIG. 19. The PET/CT images were merged and converted into MIP images, which showed uptake of the probes in the tumor and major tissues and organs at all the points of time. As shown, the uptake of the probes in the tumor gradually increased over time, to a peak at 72 hours, and then gradually decreased. ROI uptake in the tumor and major tissues and organs (heart, liver, lungs, kidneys, muscles, spleen and bones) was analyzed and plotted against time. The plotted curves indicated that [⁸⁹Zr]Zr-DFO-ABDB3 and [⁸⁹Zr]Zr-DFO-ABDB6 had good signal-to-noise ratio (SNR) characteristics and stability in vivo, as shown in FIG. 20 and FIG. 21. The in vitro biodistribution data additionally revealed the in vivo distribution of the [⁸⁹Zr]Zr-DFO-ABDB3 and [⁸⁹Zr]Zr-DFO-ABDB6 probes in the tumor and major tissues and organs, as shown in FIG. 22. These immuno-PET imaging results of [⁸⁹Zr]Zr-DFO-ABDB3 and [⁸⁹Zr]Zr-DFO-ABDB6 further confirmed the enrichment of the CD70-specific nanobody fusion proteins ABDB3 and ABDB6 in the tumor. Furthermore, the results of IHC staining of a tumor with the CD70-specific antibody E3Q1A confirmed the expression of CD70 in the tumor, as shown in FIG. 23.

### 6) RCC Diagnosis Tests Based on Immuno-PET Imaging Using [¹⁸F]F-B3 and [¹⁸F]F-B6

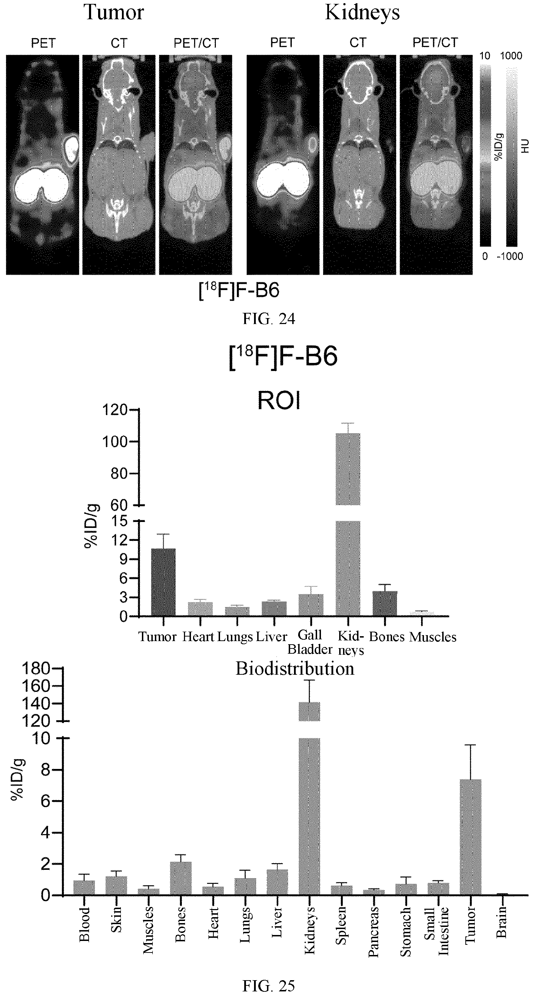

The same operations as in step 2) above were performed, and the test results are shown in FIG. 24. As indicated by PET/CT images taken 30 minutes after the CD70-specific nanobody probe [¹⁸F]F-B6 was injected into RCC models (No. 62 PDX), the probe exhibited considerable uptake in tumor tissue. As shown in the ROI uptake data of FIG. 25, high uptake occurred both in the tumor 30 minutes after injection, and in excretory organs (the kidneys and gallbladder). The *in vitro* distribution data of FIG. 25 additionally confirmed enrichment of the probes in the tumor.

The above embodiments are presented to help those of ordinary skill in the art understand and practice the present invention. Apparently, one familiar with the art can readily make various changes to those embodiments and apply the general principles disclosed herein to other embodiments without paying any creative effort. Accordingly, it is intended that the present invention is not limited to the embodiments disclosed herein, and any and all modification and changes made in light of the present disclosure without departing from the spirit of the present invention are embraced within the scope thereof.

## Claims

1. A CD70-specific nanobody, wherein the CD70-specific nanobody is B3 or B6;
wherein the B3 has an amino acid sequence as shown in SEQ ID NO. 1, and the B6 has an amino acid sequence as shown in SEQ ID NO. 3.

2. The CD70-specific nanobody according to claim 1, wherein the B3 has a gene sequence as shown in SEQ ID NO. 2 and the B6 has a gene sequence as shown in SEQ ID NO. 4.

3. Use of the CD70-specific nanobody according to claim 1 or claim 2 in the manufacture of a CD70-specific nanobody fusion protein.

4. A CD70-specific nanobody fusion protein, wherein the CD70-specific nanobody fusion protein is ABDB3 or ABDB6;
wherein the ABDB3 has an amino acid sequence as shown in SEQ ID NO. 5, and the ABDB6 has an amino acid sequence as shown in SEQ ID NO. 7.

5. The CD70-specific nanobody fusion protein according to claim 4, wherein the ABDB3 has an amino acid sequence as shown in SEQ ID NO. 6 and the ABDB6 has an amino acid sequence as shown in SEQ ID NO. 8.

6. Use of the CD70-specific nanobody according to any of claims 1 to 2 or the CD70-specific nanobody fusion protein according to any of claims 4 to 5 in the manufacture of a CD70-specific theranostic molecular imaging probe.

7. A CD70-specific theranostic molecular imaging probe, wherein the probe comprises a tumor-targeting group and a radionuclide;
wherein the tumor-targeting group is selected from the CD70-specific nanobodies of any of claims 1 to 2 or the CD70-specific nanobody fusion proteins of any of claims 4 to 5.

8. The CD70-specific theranostic molecular imaging probe according to claim 7, wherein the radionuclide is selected from ^{6g}Ga, ¹⁸F, ⁶⁴Cu or ⁸⁹Zr,
wherein when the radionuclide is selected from ⁶⁸Ga, ⁶⁴Cu or ⁸⁹Zr, the probe further comprises a chelating agent selected from *p*-SCN-Bn-NOTA or*p*-SCN-Bn-Deferoxamine.

9. The CD70-specific theranostic molecular imaging probe according to claim 7 or claim 8, wherein the probe is any one of the followings: ⁶⁸Ga-labeled monovalent nanobody probes [⁶⁸Ga]Ga-NOTA-B3 and [⁶⁸Ga]Ga-NOTA-B6, ⁶⁸Ga-labeled nanobody fusion protein probes [⁶⁸Ga]Ga-NOTA-ABDB3 and [⁶⁸Ga]Ga-NOTA-ABDB6, ¹⁸F-labeled monovalent nanobody probes [¹⁸F]F-B3 and [¹⁸F]F-B6, and ⁸⁹Zr-labeled nanobody fusion protein probes [⁸⁹Zr]Zr-DFO-ABDB3 and [⁸⁹Zr]Zr-DFO-ABDB6.

10. The CD70-specific theranostic molecular imaging probe according to claim 8, wherein when the probe comprises a tumor-targeting gene, the chelating agent and the radionuclide ⁶⁸Ga, ⁶⁴Cu or ⁸⁹Zr, the preparation method of the probe comprising the steps of:
modifying the tumor-targeting gene with the chelating agent, obtaining a modified nanobody; and then labeling the modified nanobody with the radionuclide ⁶⁸Ga, ⁶⁴Cu or ⁸⁹Zr, thereby obtaining the probe;
alternatively, wherein when the probe comprises a tumor-targeting gene and the radionuclide ¹⁸F, the preparation method thereof comprising the steps of:
labeling a precursor with the radionuclide ¹⁸F, which is a small-molecule compound, obtaining a ¹⁸F-labeled precursor;
preparing a nanobody randomly conjugated with DBCO; and
obtaining the probe from a click chemistry reaction between the ¹⁸F-labeled precursor and the nanobody randomly conjugated with DBCO.
